# EUROPEAN PATENT APPLICATION

(11) **EP 2 591 747 A1**
(43) Date of publication of application: **15.05.2013**
(21) Application number: 12191630.8
(22) Date of filing: 07.11.2012
(51) Int. Cl.: A61C 1/08, A61C 8/00

(54) **System for implanting an artificial tooth root**

(30) Priority: 14.11.2011 JP 2011248452
(71) Applicant: GC Corporation, Bunkyo-ku Tokyo 113-0033 (JP)
(72) Inventor: Fujii, Naoto, Tokyo, 174-8585 (JP); Yoshihara, Hirotaka, Tokyo, 174-8585 (JP)
(74) Representative: Smart, Peter John

(57) **Abstract**

The present invention provides a system for embedding an artificial tooth root whereby holes of various depths can be bored into an alveolar bone more stably and kinds of drills and kinds of spacers to be included herein can be reduced. The system for embedding an artificial tooth root comprises: two kinds of dental drills to bore a hole into the alveolar bone which have predetermined different lengths and have a predetermined relation; and one to three kinds of spacers to be attached to the dental drills, for combinations of an artificial tooth root and a surgical guide, the artificial tooth root being selected from a group of artificial tooth roots formed of two to five kinds of artificial tooth roots, and the surgical guide being selected from a group of surgical guides formed of one to three kinds of surgical guides.

## Description

### Technical Field

The present invention relates to a system for embedding an artificial tooth root, which is employed to bore into an alveolar bone a hole to be embedded with an artificial tooth root in placing a dental prosthesis of a single tooth, a series of teeth or a denture to fill up a defective site in the tooth row by using the artificial tooth root (implant).

### Background Art

When there is a defect in part of teeth in a tooth row, some measures to repair this defective site are often taken, and using a dental prosthesis structure that includes an artificial tooth root (a so-called implant) is one of them. This is used by creating a predetermined hole in an alveolar bone at the defective part in the tooth row; embedding an artificial tooth root in this hole; and fixating a dental prosthesis on the top end of the artificial tooth root. This does not require use of a clasp or the like, and therefore gives pleasant appearances compared with the so-called conventionally available artificial tooth. It is also better in that it can be placed stably in an oral cavity.

In using the dental prosthesis structure that includes an artificial tooth root, a hole is created in the alveolar bone and an artificial tooth root is embedded in this hole. However, as each patient has a different condition of his/her alveolar bone, multiple kinds of artificial tooth roots having different lengths are usually prepared and an artificial tooth root in a proper length is selected to be embedded in the hole. The hole to be embedded with the artificial tooth root is created by a dental drill in accordance with thus selected artificial tooth root. At this time, if the hole bored does not have a desired depth, the artificial tooth root sometimes cannot be placed firmly; or when it is placed, it sometimes cannot fixate a dental prosthesis stably on its top end. On the other hand, boring a hole deeper than intended may damage other tissues. Therefore, in embedding an artificial tooth root, it is important to bore a hole to be embedded with the artificial tooth root at an accurate position and depth.

These days, before embedding an artificial tooth root, an x-ray CT (Computed Tomography) is used to take images of various fault planes in the alveolar bone area to be embedded with the artificial tooth root, thereby obtaining detailed information on the alveolar bone. Then the direction or position in which the artificial tooth root is embedded in the alveolar bone is determined by simulation. In order to bore a hole to be embedded with an artificial tooth root at an accurate position, a guide (sometimes called a surgical guide or a surgical stent) for boring a hole for an artificial tooth root is used, the guide being formed based on the data obtained through the above CT. Such a guide has a guide hole to guide a drill used for boring a hole in the alveolar bone, and is placed over a tooth row when used. At this time, a predetermined space is arranged between the guide and the face of the alveolar bone to be bored, in order to dissipate the heat generated in boring the hole.

On the other hand, in order to regulate the depth of a bored hole to a desired one, a stopper is attached to a predetermined position of a drill. Thereby, the stopper is caught by the guide when the drill reaches the depth of the hole to be bored, which enables prevention of the drill from going deeper. Patent Document 1 discloses a stopper, and a technique that the fixation and movement of the stopper can be changed to a predetermined position of a drill by using a screw and that the depth of a hole to be bored by the drill can be freely changed.

In addition, Patent Document 2 discloses a dental drill which is provided with a stopper in a flange shape (in a disc shape) in order to regulate the depth of a bored hole to a desired one, the stopper being arranged at a predetermined position in a spindle part of the drill and integrated with the spindle part. Thereby as well, the stopper is caught by the guide when the drill reaches the depth of the hole to be bored, which enables prevention of the drill from going deeper.

Further, there is also a drill, like the one described in Patent Document 2, which is provided with a stopper in a flange shape integrated with a spindle part of the drill, wherein a detachable spacer is provided to the stopper. This makes it possible to regulate the depth of the drill entering the hole in multiple ways even with one dental drill by changing the size of the spacer.

### Citation List

### Patent Documents

Patent Document 1: Japanese Patent Application Laid-Open (JP-A) No. 2009-279147
Patent Document 2: JP-A No. 2005-518834

### Summary of the Invention

### Problems to be Solved by the Invention

According to the invention described in Patent Document 1, there is an advantage that the position of the stopper can be changed freely. However, the positional adjustment of the stopper tends to be complicated, and the stopper is likely to move if it is not fixated firmly by the screw. In addition, according to the invention described in Patent Document 2, the stopper is fixated and thus the above problem can be solved. However, a number of drills in the same diameter each having a stopper at a different position need to be prepared. Further, a drill with a spacer can bore a deep hole by arranging a long distance from a front end of the drill to the stopper. On the other, when boring a shallow hole, it is necessary to attach a large spacer in an axial direction of the drill, which tends to cause an unstable axis. Moreover, when such a long drill is inserted into an oral cavity of a patient, the patient needs to open his/her mouth widely.

Accordingly, an object of the present invention is to provide a system (i.e. a set of dental components) for embedding an artificial tooth root whereby holes of various depths can be bored into an alveolar bone more stably and kinds of drills and kinds of spacers to be included herein can be reduced.

### Means for Solving the Problems

The present invention will be described below. In order to make the present invention easy to understand, reference numerals given in the accompanying drawings are shown here in parentheses. However, the present invention is not limited to the embodiments shown in the drawings.

A first aspect of the present invention is a system (30) for embedding an artificial tooth root, comprising two kinds of dental drills (34, 35, D₁, D₂) to bore a hole into an alveolar bone, and a group of spacers comprising one to three kinds of spacers (31 to 33, S₁ to S₃) to be attached to the dental drills, for combinations of an artificial tooth root and a surgical guide, the artificial tooth root being selected from a group (40) of artificial tooth roots formed of two to five kinds of artificial tooth roots (41 to 45, F₁ to F₅), and the surgical guide being selected from a group (50) of surgical guides formed of one to three kinds of surgical guides (51 to 53, G₁ to G₃), wherein with an integer in a range of 2≤x≤5, the group of artificial tooth roots assumed is constituted by an artificial tooth root which has a length of M₁ from its front end to its portion to be positioned at the face of the alveolar bone when embedded, and an artificial tooth root which has a length represented by Mₓ=M₁+(x-1) ·p from its front end to its portion to be positioned at the face of the alveolar bone when embedded, thereby comprising x kinds of artificial tooth roots of M₁ to Mₓ; with an integer in a range of 1≤y≤3, the group of surgical guides assumed comprises y kinds of surgical guides of N₁ to N_{y} having a space of N_{y}=N₁+ (y-1) ·p from the surgical guide to the face of the alveolar bone to be embedded with the artificial tooth root, in a posture of being fitted in an oral cavity; the two kinds of dental drills each comprises a stick-shaped drill main body (34a, 35a) provided with a drill blade, and a protrusion portion (34b, 35b) arranged in a manner protruding from one part of the side face of the drill main body; the spacer is in a tubular shape, allowing the drill main body to penetrate inside the tubular shape and not allowing the portion of the drill provided with the protrusion portion to penetrate thereinside; with an integer in a range of 1≤z≤3, a group of the spacers comprises z kinds of spacers of Q₁ to Q_{z} provided with a spacer portion having a length of Q_{z}=z ·p in an axial direction of the tubular-shaped spacer; the number z is defined from the x and the y as: z=3 in a case of x+y=8; z=2 in a case of 6≤x+y≤7; and z=1 in a case of 2≤x+y≤5; and when a distance between a face of the surgical guide which faces the alveolar bone face and its face opposite thereto is defined as T, a distance L₁ is L₁=T+N₁+M₁+z ·p from the front end to the protrusion portion of one (34, D₁) of the two kinds of dental drills, and a distance L₂ is L₂=T+N_{y}+Mₓ from the front end to the protrusion portion of the other dental drill (35, D₂).

A second aspect of the present invention is the system for embedding an artificial tooth root according to the first aspect, wherein the L₂ is 30 mm or less; and a relation between the L₂ and the L₁ is configured in a range satisfying L₂-L₁=2 ·p.

### Effects of the Invention

According to the present invention, holes of various depths can be bored into an alveolar bone more stably, and kinds of drills and kinds of spacers to be included can be reduced.

### Brief Description of the Drawings

Fig. 1 is a view illustrating a configuration of a dental prosthesis structure.
Fig. 2 is a view showing a flow of process for applying the dental prosthesis structure.
Fig. 3 is a view illustrating a surgical guide.
Fig. 4 is a view showing a system 30 for embedding an artificial tooth root according to one embodiment.
Fig. 5 is a view showing examples of a combination of a dental drill and a spacer.
Fig. 6 is a view showing kinds of artificial tooth roots assumed by the system 30 for embedding an artificial tooth root.
Fig. 7 is a view showing kinds of surgical guides assumed by the system 30 for embedding an artificial tooth root.
Fig. 8 is a view illustrating a positional relation among each element.

### Modes for Carrying Out the Invention

The functions and benefits of the present invention described above will be apparent from the following modes for carrying out the invention. Hereinafter, the present invention will be described based on the embodiments shown in the drawings. However, the invention is not limited to these embodiments.

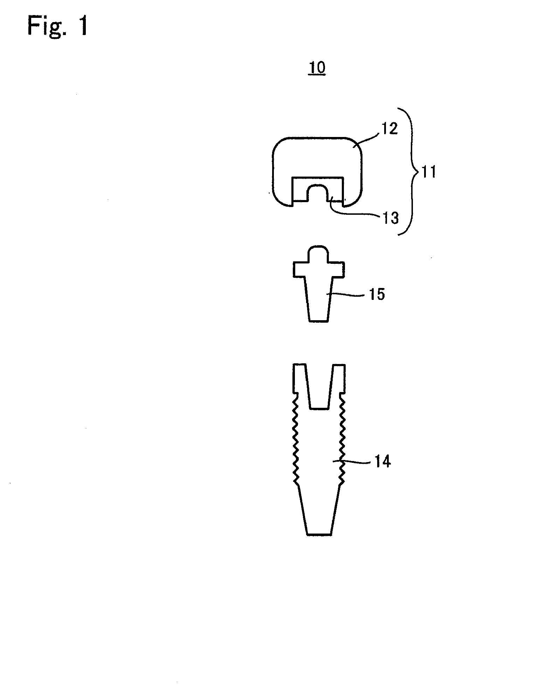

First, a configuration of a dental prosthesis structure in which an artificial tooth root is included will be described. Fig. 1 schematically shows a configuration of a dental prosthesis structure 10 as one example. The dental prosthesis structure 10 comprises a dental prosthesis 11, an artificial tooth root 14, and a connecting member 15. Herein, the dental prosthesis 11 includes an artificial tooth crown 12 and a connection receiving portion 13.

The artificial tooth crown 12 is a main part of the dental prosthesis 11, and actually compensates for a defective part in a tooth row. Accordingly, the artificial tooth crown 12 is in a shape modeled after a tooth, thus reproducing a shape and texture of the tooth.
The connection receiving portion 13 is arranged in a part on the alveolar bone side of the artificial tooth crown 12 in the dental prosthesis 11, and connects with the connecting member 15 in a manner accommodating the connecting member 15 thereinto.
The artificial tooth root 14 is also called an implant or an implant body, and is a member which is embedded in the alveolar bone and properly fixates the dental prosthesis structure 10 in the oral cavity.
The connecting member 15 is disposed between the dental prosthesis 11 and the artificial tooth root 14 and connects these. More specifically, one part of the connecting member 15 is inserted into the artificial tooth root 14 to be fixated, and the other part of the connecting member 15 is inserted into the connection receiving portion 13 of the dental prosthesis 11 to be fixated.

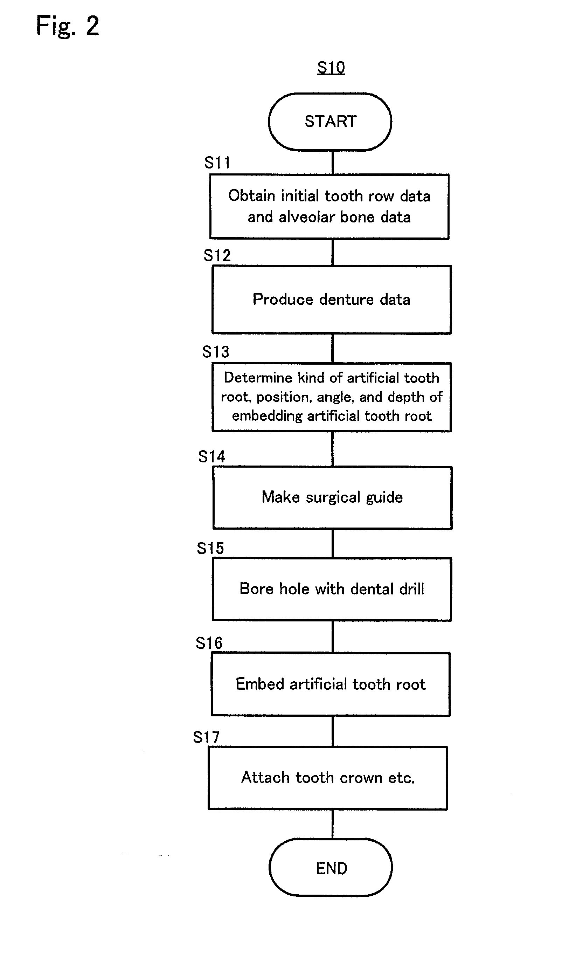

Next, an outline of a flow for fixating the dental prosthesis structure 10 in the oral cavity will be described. Fig. 2 shows in a flowchart a method S10 of forming a dental prosthesis structure as one example. The method S10 of forming a dental prosthesis structure is carried out through the steps S11 to S17 for example. Each of the steps will be described below.

In the step S11, the following are obtained: initial tooth row data of a patient containing a defective part, which is three-dimensional tooth row data; and three-dimensional alveolar bone data of the patient. Herein, the initial tooth row data can be obtained by first obtaining a tooth row model of the patient which contains the defective part, and then turning a surface shape of the tooth row model into three-dimensional data using a three-dimensional shape measuring apparatus or the like. Examples of the three-dimensional shape measuring apparatus include: a non-contact three-dimensional digitizer; a non-contact three-dimensional contour reader; and a non-contact laser scanner. As for the defective part, three-dimensional data of a gum surface (residual ridge) are obtained.
On the other hand, the alveolar bone data can be obtained using a CT imaging device which scans with an x-ray beam to obtain a tomographic image of an internal organ of the human body. With the CT imaging device, it is possible to take images of the alveolar bone of the patient and thereby obtain a plurality of two-dimensional CT images with different cross-sectional positions. These images are synthesized and thereby turned into three-dimensional data. In this way, the three-dimensional alveolar bone data can be obtained.

In the step S12, three-dimensional dental prosthesis data are produced so as to match the defective part of the initial tooth row data based on the initial tooth row data and the alveolar bone data that have been obtained. To produce the dental prosthesis data, for example the dental prosthesis data to be applied to the defective part is computed by a computing device such as a computer, from the obtained initial tooth row data and alveolar bone data based on the predetermined procedures by a given program. A specific method of producing the dental prosthesis data is not particularly limited, and thus a known method may be employed. An example may be selecting suitable dental prosthesis data from a number of dental prosthesis data as references that have been made into a database, and determining the configuration for example by enlarging, reducing, or deforming the selected dental prosthesis data while considering the shape of the patient's remaining teeth. Additionally, if the patient uses another dental prosthesis, the shape of this dental prosthesis may be taken into consideration.

Subsequently, the step S13 will be described. In the step S13, the kind of artificial tooth root to be used is determined based on the initial tooth row data and the alveolar bone data that have been obtained and the dental prosthesis data that have been produced; and the position, angle, depth etc. at which to embed the artificial tooth root is determined using these data. Thereby, three-dimensional data thereof are produced. To produce such data, data of an artificial tooth root to be applied is computed by a computing device such as a computer, from the obtained initial tooth row data, alveolar bone data, and the produced dental prosthesis data based on the predetermined procedures by a given program. The kind of artificial tooth root to be used can be obtained for example by selecting the artificial tooth root data that most suit the computation result, from a number of artificial tooth roots stored in a database.
On the other hand, the position, angle and depth at which to embed the artificial tooth root are computed based on the obtained initial tooth row data and alveolar bone data. In this computation, the shape of the alveolar bone, nerves, etc. are taken into consideration to compute a configuration which is as suitable as possible for embedding the artificial tooth root. This computation can also be obtained by the computing device such as a computer based on the predetermined procedures by a program.

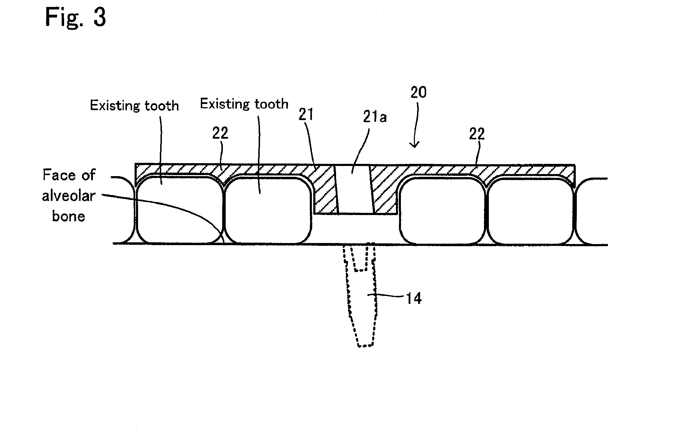

The step S14 is a step of making a surgical guide, which is a guide member for embedding the artificial tooth root precisely, based on the shape of the artificial tooth root, the position at which to place the artificial tooth root, and other aspects determined in the step S13. Fig. 3 shows a schematic view illustrating a configuration of a surgical guide 20. The surgical guide 20 is configured to have a guide portion 21 and a support portion 22.
The guide portion 21 is a portion to be disposed in a defective part in the tooth row, and has a guide hole 21a arranged at an angle and a position that allow it to communicate with a hole to be embedded with the artificial tooth root 14.
The support portion 22 is a portion that extends from the guide portion 21, and has a shape in accordance with the surface of a plurality of existing teeth adjacent to the defective part.
That is, the guide portion 21 is placed in the defective part in the oral cavity of a patient, and the support portion 22 is placed over the existing teeth adjacent to the defective part. Thereby, the guide portion 21 is placed stably in the defective part. In addition, the guide hole 21a is automatically placed at an angle and a position that allow it to communicate with a hole to be bored into the alveolar bone.
Accordingly, a dentist can precisely create a hole to be embedded with an artificial tooth root by placing the surgical guide 20 in the oral cavity, inserting a dental drill into the guide hole 21a, and boring a hole in the alveolar bone while guiding the dental drill. At this time, a stopper or a spacer placed thereat is used to prevent the dental drill from going deeper than necessary, thereby ensuring the safety. The details will be given later.
The support portion 22 described herein is configured to overlay the existing teeth, and thus is supported by the teeth. However, the supporting system is not limited to this. It may be supported for example by the alveolar mucosa or by the alveolar bone.

Such a surgical guide can be made in a conventional manner and is not particularly limited. However, in the present embodiment, the guide hole 21a provided to the surgical guide is based on the position and the shape of the artificial tooth root obtained in the above step S13. The surgical guide 20 can be made by using a surgical guide manufacturing apparatus equipped with a cutting device for example in the following way.

First, the surgical guide manufacturing apparatus obtains the data generated in each of the above described steps. Then, a block as a material is sent to a part where cutting processing is performed. At this time, the material of the surgical guide may be an ordinarily employed one, an example of which may be an acrylic resin. The block placed in the part where the cutting processing is performed is cut based on the above obtained data. Thereby, the guide portion 21, the support portion 22, and the guide hole 21a are formed. Herein, an example is given that the surgical guide is made through cutting processing. In addition to this, it may also be made through casting using a mold. Further, if CAD/CAM data have been produced, the CAD/CAM may be used to design and fabricate the surgical guide.

As shown in Fig. 2, the step S15 is a step of boring a hole into the alveolar bone by using a dental drill. Details are as follows. First, the surgical guide 20 made in the above way is placed over the tooth row as shown in Fig. 3. At this point, as can be understood from the Fig. 3, a predetermined space is created between the surgical guide 20 and the face of the alveolar bone. Next, a dental drill is inserted into the guide hole 21a of the surgical guide 20; and with the dental drill guided, a desired hole is created into the alveolar bone. At this time, the protrusion portion of the dental drill that functions as a stopper, or the spacer gets caught at the guide hole 21a, being unable to enter the guide hole 21a, thereby preventing the dental drill from going further deeper. The dental drill is removed after the desired hole is created, and the surgical guide is released from the tooth row.

As shown in Fig. 2, the step S16 is a step of embedding the artificial tooth root 14. The artificial tooth root 14 has a screw shape on its periphery as also apparent from Fig. 1. This screw shape is utilized in inserting the artificial tooth root into the hole created in the alveolar bone and screwing it. Thereby, the artificial tooth root 14 can be strongly fixated in the alveolar bone.

The step S17 is a step of attaching a tooth crown etc. In specific, the connecting member 15 is attached to the artificial tooth row 14 fixated in the alveolar bone, and then the dental prosthesis 11 is placed on the connecting member 15. Thereby, the whole dental prosthesis structure 10 is placed in the oral cavity, and fulfills its function.

The system for embedding an artificial tooth root of the present invention is employed in fixating a dental prosthesis structure described above in the oral cavity. Particularly, it is employed in the step of boring a hole into the alveolar bone by using a dental drill, which has been described in the step S15. Descriptions of one embodiment of the present invention will be given below.

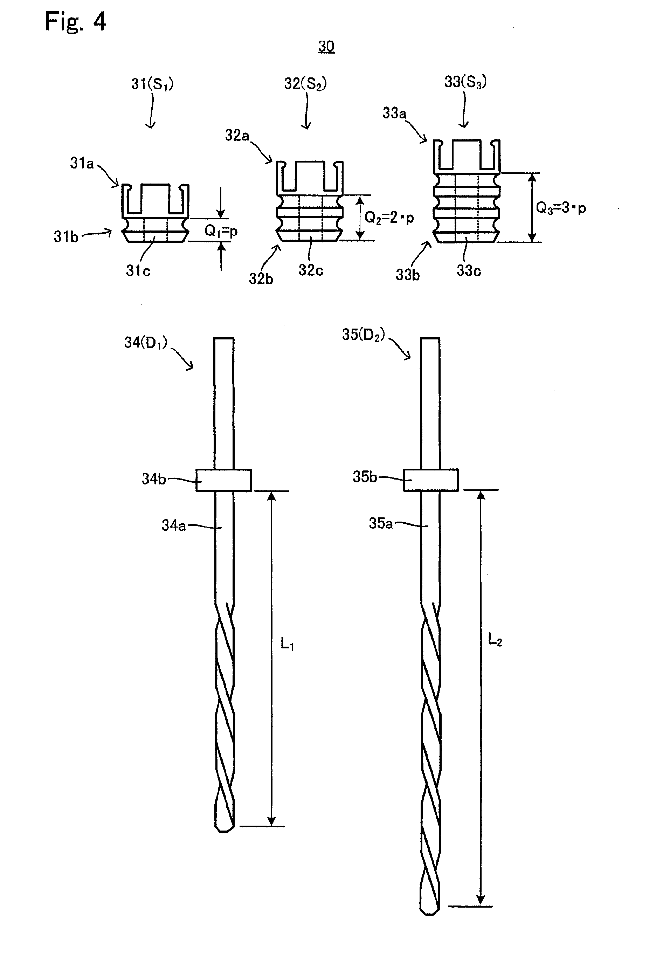

Fig. 4 is a view showing the system 30 for embedding an artificial tooth root according to one embodiment, and shows each member to constitute the system 30 for embedding an artificial tooth root. The system 30 for embedding an artificial tooth root according to the present embodiment comprises: a first dental drill 34; a second dental drill 35; and a first spacer 31, a second spacer 32, and a third spacer 33 that constitute a group of spacers.

The first dental drill 34 has a drill main body 34a and a protrusion portion 34b. The drill main body 34a is the same as that of an ordinary dental drill. The protrusion portion 34b is arranged in a side part of the drill main body 34a where a drill blade is not formed, in a manner protruding from the side part. The protrusion portion 34b functions as a portion to engage with the spacer (31, 32, 33); and when the spacer (31, 32, 33) is not provided, it functions as a stopper.
Further, as shown in Fig. 4, the distance from the front end to the protrusion portion 34b of the first dental drill 34 is L₁. The size of the distance L₁ will be explained later. Herein, the "front end of the drill" refers to the front end of a portion of the drill that can create a hole of a target diameter. Usually, the tip of a drill is tapered in a cone shape, but a hole of a target diameter cannot be created by the conically-shaped portion, which thus is excluded herein. The same shall apply hereinafter.
The first dental drill 34 may be represented as D₁ to make descriptions thereof easy.

The second dental drill 35 has a drill main body 35a and a protrusion portion 35b. The drill main body 35a is the same as that of an ordinary dental drill. The protrusion portion 35b is arranged in a side part of the drill main body 35a where a drill blade is not formed, in a manner protruding from the side part. The protrusion portion 35b functions as a portion to engage with the spacer (31, 32, 33); and when the spacer (31, 32, 33) is not provided, it functions as a stopper.
Herein, the distance from the front end to the protrusion portion 35b of the second dental drill 35 is L₂. L₂ is larger than L₁. The distance L₂ will be explained later. The second dental drill 35 may be represented as D₂ to make descriptions thereof easy.

The first spacer 31 is one of the spacers to constitute the group of spacers, and functions as a stopper. As can be seen from Fig. 4, the first spacer 31 comprises an attachment portion 31a and a spacer portion 31b. As a whole, the first spacer 31 is a cylindrical member having an axis in the top and bottom direction of the drawing sheet of Fig. 4; and has a hollow portion 31c thereinside (the hollow portion is shown by a broken line with the first spacer seen through.). The hollow portion 31c is configured to allow the drill main body 34a and the drill main body 35a to penetrate therethrough and not to allow the protrusion portions 34b and 35b to penetrate therethrough. The attachment portion 31a has claw-shaped members extending in the axial direction of the cylindrical member and arranged at a predetermined interval along the outer perimeter of the cylindrical member. The spacer portion 31b is formed to have a thickness of Q₁=p in the axial direction of the cylindrical shape. The first spacer 31 may be represented as S₁ to make descriptions thereof easy.

The second spacer 32 is one of the spacers to constitute the group of spacers, and functions as a stopper. As can be seen from Fig. 4, the second spacer 32 comprises an attachment portion 32a and a spacer portion 32b. As a whole, the second spacer 32 is a cylindrical member having an axis in the top and bottom direction of the drawing sheet of Fig. 4; and has a hollow portion 32c thereinside (the hollow portion is shown by a broken line with the second spacer seen through.). The hollow portion 32c is configured to allow the drill main body 34a and the drill main body 35a to penetrate therethrough and not to allow the protrusion portions 34b and 35b to penetrate therethrough. The attachment portion 32a has claw-shaped members extending in the axial direction of the cylindrical member and arranged at a predetermined interval along the outer perimeter of the cylindrical member. The spacer portion 32b is formed to have a thickness of Q₂=2 ·p in the axial direction of the cylindrical shape. The second spacer 32 may be represented as S₂ to make descriptions thereof easy.

The third spacer 33 is one of the spacers to constitute the group of spacers, and functions as a stopper. As can be seen from Fig. 4, the third spacer 33 comprises an attachment portion 33a and a spacer portion 33b. As a whole, the third spacer 33 is a cylindrical member having an axis in the top and bottom direction of the drawing sheet of Fig. 4; and has a hollow portion 33c thereinside (the hollow portion is shown by a broken line with the third spacer seen through.). The hollow portion 33c is configured to allow the drill main body 34a and the drill main body 35a to penetrate therethrough and not to allow the protrusion portions 34b and 35b to penetrate therethrough. The attachment portion 33a has claw-shaped members extending in the axial direction of the cylindrical member and arranged at a predetermined interval along the outer perimeter of the cylindrical member. The spacer portion 33b is formed to have a thickness of Q₃=3 ·p in the axial direction of the cylindrical shape. The third spacer 33 may be represented as S₃ to make descriptions thereof easy.

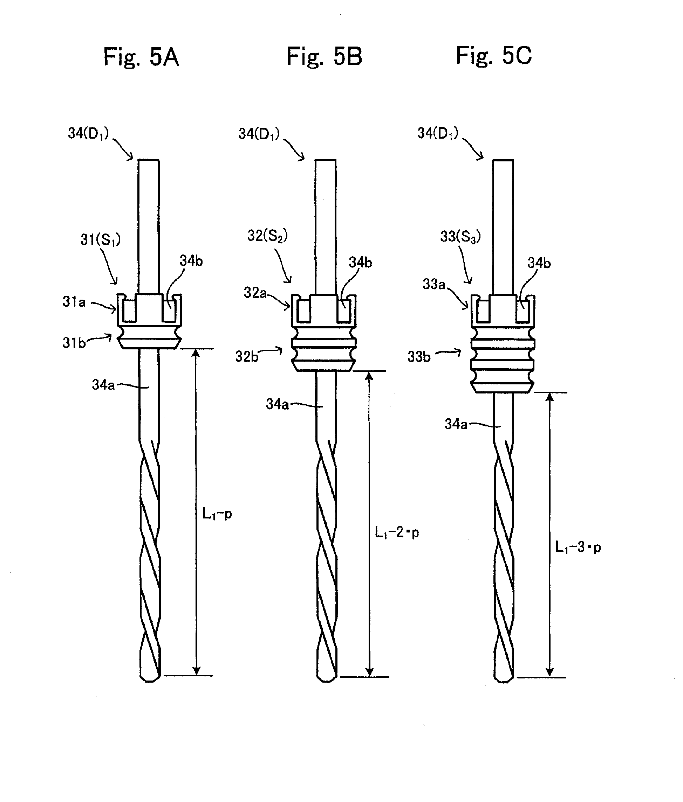

The first dental drill 34 and the second dental drill 35 as above may be respectively combined with the first spacer 31, the second spacer 32, and the third spacer 33 for example in the following manner. Combinations of the first dental drill 34 with the first spacer 31, with the second spacer 32, and with the third spacer 33 will be illustrated as examples, which are shown in Fig. 5. Fig. 5A shows a combination of the first dental drill 34 with the first spacer 31. Fig. 5B shows a combination of the first dental drill 34 with the second spacer 32. Fig. 5C shows a combination of the first dental drill 34 with the third spacer 33.

As can be understood from Fig. 5A, the drill main body 34a is inserted into the hollow portion 31c of the first spacer 31 (see Fig. 4) in a direction from the front end side of the first dental drill 34 to be passed therethrough; and the attachment portion 31a is engaged with the protrusion portion 34b of the first dental drill 34. Thereby, they are fixated with each other. In this way, the first dental drill 34 can be attached to the first spacer 31.
With the first spacer 31 attached to the first dental drill 34, the distance from the front end of the first dental drill 34 to the first spacer 31 is L₁-p, as also shown in Fig. 5A.

Likewise as can be seen from Fig. 5B, the second spacer 32 can be attached to the first dental drill 34. At this time, the distance from the front end of the first dental drill 34 to the second spacer 32 is L₁-2 ·p.
Further, as can be seen from Fig. 5C, the third spacer 33 can be attached to the first dental drill 34. At this time, the distance from the front end of the first dental drill 34 to the third spacer 33 is L₁-3 ·p.

Herein, the combinations of the first dental drill 34 with each of the spacers (31, 32, 33) have been illustrated. The combinations of the second dental drill 35 with each of the spacers (31, 32, 33) are also the same. That is, in the combination of the second dental drill 35 with the first spacer 31, the distance from the front end of the second dental drill 35 to the first spacer 31 is L₂-p. Likewise, in the combination of the second dental drill 35 with the second spacer 32, the distance from the front end of the second dental drill 35 to the second spacer 32 is L₂-2 ·p. In the combination of the second dental drill 35 with the third spacer 33, the distance from the front end of the second dental drill 35 to the third spacer 33 is L₂-3 ·p.

This system 30 for embedding an artificial tooth root can create a hole into the alveolar bone based on the combinations of five kinds of artificial tooth roots 41 to 45 having different lengths with three kinds of surgical guides 51 to 53 having different sizes of the space between the surgical guide and the face of the alveolar bone. Detailed descriptions will be given below.

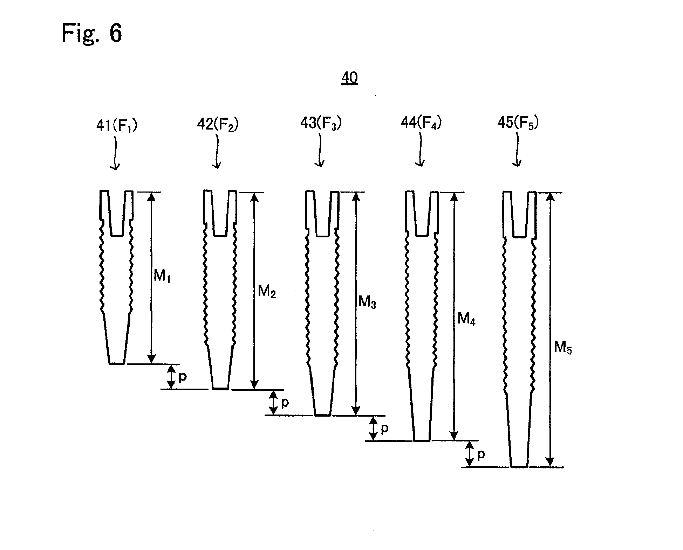

Fig. 6 shows artificial tooth roots 41 to 45 constituting a group 40 of artificial tooth roots that can be assumed in the present embodiment. These five artificial tooth roots are configured to differ from one another in length by a degree of "p". That is, in the artificial tooth root 41, the distance is M₁ from its front end on the side to be embedded deeper into the alveolar bone (which will be written as a "front end" hereinafter) to its portion at the same position as the face of the alveolar bone when embedded (the distance will be referred to as an "embedding length".). The embedding length of the artificial tooth root 42 is M₂, which is M₁+p (M₂= M₁+p). Likewise, the embedding length of the artificial tooth root 43 is M₃, which is M₁+2 ·p (M₃= M₁+2 ·p). The embedding length of the artificial tooth root 44 is M₄, which is M₁+3 ·p (M₄= M₁+3 ·p). The embedding length of the artificial tooth root 45 is M₅, which is M₁+4 ·p (M₅= M₁+4 ·p).
To make descriptions thereof easy, the artificial tooth root 41 may be represented as F₁; the artificial tooth root 42 may be represented as F₂; the artificial tooth root 43 may be represented as F₃; the artificial tooth root 44 may be represented as F₄; and the artificial tooth root 45 may be represented as F₅.

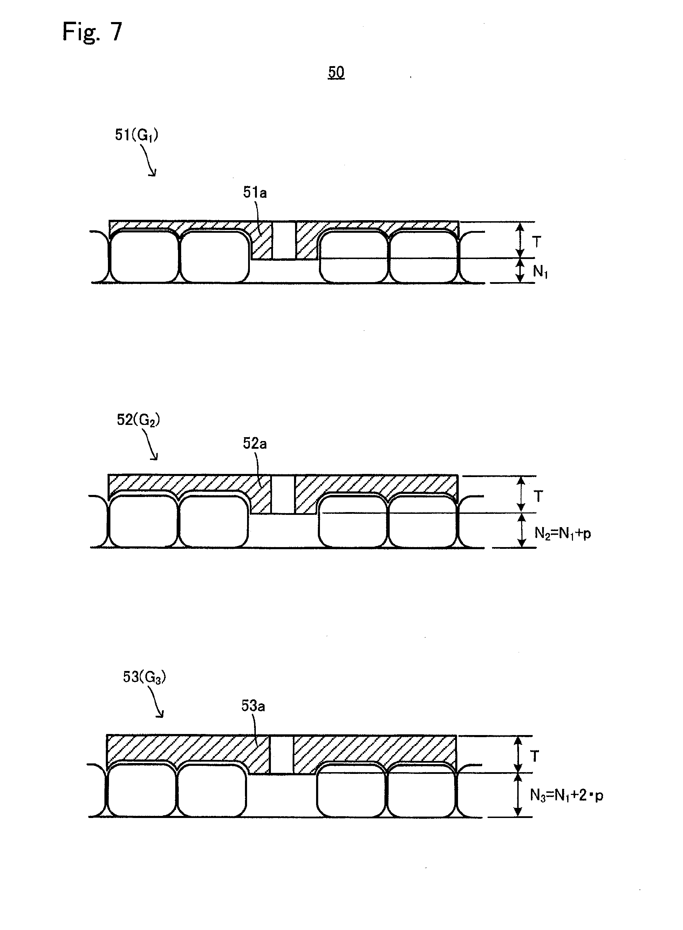

Fig. 7 shows surgical guides 51 to 53 that constitute a group 50 of surgical guides that can be assumed in the present embodiment. These three surgical guides are configured to have a thickness of T and to differ from one another in terms of the space between them and the face of the alveolar bone by a degree of "p". That is, as can be understood from Fig. 7, the surgical guide 51 has a space N₁ between its guide portion 51a and the face of the alveolar bone in a posture of being fitted in the oral cavity. Likewise, the surgical guide 52 has a space N₂ between its guide portion 52a and the face of the alveolar bone in a posture being fitted in the oral cavity, N₂ being N₁+p (N₂= N₁+p) The surgical guide 53 has a space N₃ between its guide portion 53a and the face of the alveolar bone in a posture being fitted in the oral cavity, N₃ being N₁+2 ·p (N₃= N₁+2 ·p).
To make descriptions thereof easy, the surgical guide 51 may be represented as G₁; the surgical guide 52 may be represented as G₂; and the surgical guide 53 may be represented as G₃.

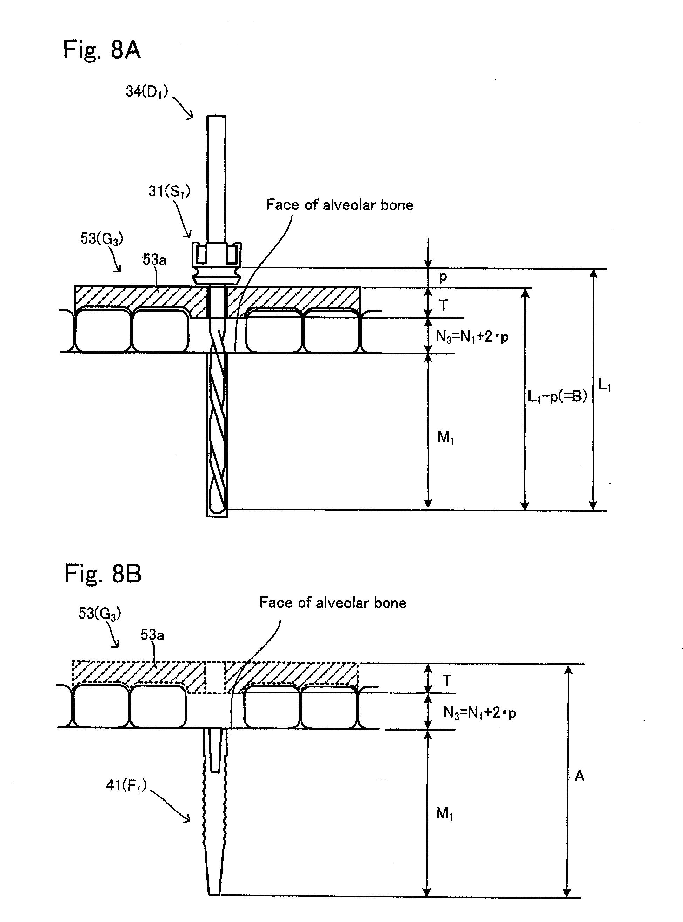

According to the artificial tooth roots 41 to 45 and the surgical guides 51 to 53, the combinations thereof that can be assumed are in 15 patterns as in 5x3=15. The depth of a hole to be bored into the alveolar bone varies based on these patterns of combination. Therefore, the means to regulate the depth of the hole to be bored into the alveolar bone needs to be changed in accordance with the variations. As one example, Fig. 8 shows a view (Fig. 8B) illustrating a manner of boring a hole in a case of choosing the artificial tooth root 41 (F₁) as an artificial tooth root to be embedded and the surgical guide 53 (G₃) as a surgical guide to be used in boring the hole.

As can be understood from Fig. 8B, the embedding length of the artificial tooth root 41 is M₁; the space between the face of the alveolar bone and the surgical guide 53 is N₃; and the thickness of the surgical guide 53 is T. That is, the distance is M₁+N₃+T from the front end of the artificial tooth root 41 to the face of the surgical guide 53 which is opposite to the face of the alveolar bone. In accordance with this, the first spacer 31 is fitted to the first dental drill 34 as shown in Fig. 8A, whereby it is possible to bore a hole and to also regulate the depth of the hole properly. As can be understood from Fig. 8A, with the combination of the first dental drill 34 with the first spacer 31, the distance from the front end of the drill main body to the first spacer 31 is L₁-p. By setting L₁-p as M₁+N₃+T, the spacer 31 gets caught by the surgical guide 53, functioning as a stopper and preventing boring further deeply.

In the above descriptions, one example has been introduced. Table 1 shows all patterns of combinations of the five artificial tooth roots 41 to 45 and the three surgical guides 51 to 53; and shows combinations of the dental drill and the spacer that can be adopted for those combinations of the artificial tooth roots and the surgical guides.

**[Table 1]**

| No. | Artificial tooth root | Surgical guide | Distance from the upper surface of the surgical guide to the deepest part of the hole to be bored | Relation with F₁G₁ | Case of using a dental drill D 1 | | | Case of using a dental drill D 2 | | |
|---|---|---|---|---|---|---|---|---|---|---|
| | | | | | Dental drill | Spacer | Distance from the front end of the dental drill to the lower surface of the spacer | Dental drill | Spacer | Distance from the front end of the dental drill to the lower surface of the space |
| 1 | F₁ | G₁ | T+N ₁+M₁ | - | D₁ | S₃ | L₁-3 ·p | X | | |
| 2 | F₁ | G₂ | T+N ₂+M₁ | (F₁G₁)+p | D₁ | S₂ | L₁-2 ·p | X | | |
| 3 | F₁ | G₃ | T+N ₃+M₁ | (F₁G₁)+2 ·p | D₁ | S₁ | L₁-p | X | | |
| 4 | F₂ | G₁ | T+N ₁+M₂ | (F₁G₁)+p | D₁ | S₂ | L₁-2 ·p | X | | |
| 5 | F₂ | G₂ | T+N ₂+M₂ | (F₁G₁)+2 ·p | D₁ | S₁ | L₁-p | X | | |
| 6 | F₂ | G₃ | T+N ₃+M₂ | (F₁G₁)+3 ·p | D₁ | None | L₁ | D₂ | S₃ | L₂-3 ·p |
| 7 | F₃ | G₁ | T+N ₁M₃ | (F₁G₁)+2 ·p | D₁ | S₁ | L₁-p | X | | |
| 8 | F₃ | G₂ | T+N ₂+M₃ | (F₁G₁)+3 ·p | D₁ | None | L₁ | D₂ | S₃ | L₂-3 ·p |
| 9 | F₃ | G₃ | T+N ₃+M₃ | (F₁G₁)+4 ·p | X | | | D₂ | S₂ | L₂-2 ·p |
| 10 | F₄ | G₁ | T+N ₁+M₄ | (F₁G₁)+3 ·p | D₁ | None | L₁ | D₂ | S₃ | L₂-3 ·p |
| 11 | F₄ | G₂ | T+N ₂+M₄ | (F₁G₁)+4 ·p | X | | | D₂ | S₂ | L₂-2 ·p |
| 12 | F₄ | G₃ | T+N ₃+M₄ | (F₁G₁)+5 ·p | X | | | D₂ | S₁ | L₂-p |
| 13 | F₅ | G₁ | T+N ₁+M₅ | (F₁G₁)+4 ·p | X | | | D₂ | S₂ | L₂-2 ·p |
| 14 | F₅ | G₂ | T+N ₂+M₅ | (F₁G₁)+5 ·p | X | | | D₂ | S₁ | L₂-p |
| 15 | F₅ | G₃ | T+N ₃M₅ | (F₁G₁)+6 ·p | X | | | D₂ | None | L₂ |

In Table 1, the artificial tooth roots are represented as F₁ to F₅; the surgical guides are represented as G₁ to G₃; the dental drills are represented as D₁, D₂; and the spacers are represented as S₁ to S₃.
The "distance from the upper surface of the surgical guide to the deepest part of the hole to be bored" is determined by the kinds of artificial tooth roots to be applied and the kinds of surgical guides to be used, as described above.
The "relation with F₁G₁" shows how large the "distance from the upper surface of the surgical guide to the deepest part of the hole to be bored" should be with respect to the combination of F₁ and G₁.
The "case of using a dental drill D₁" shows the kind of spacer and so on that are applicable when using D₁ in accordance with the combination of the artificial tooth root and the surgical guide.
The "case of using a dental drill D₂" shows the kind of spacer and so on that are applicable when using D₂ in accordance with the combination of the artificial tooth root and the surgical guide.
In addition, when the drill cannot handle the case with any of the spacers, it is indicated by "×".

As can be understood from Table 1, in the present embodiment, the distance from the front end of the artificial tooth root to be embedded to the upper surface of the surgical guide (the distance equivalent to A shown in Fig. 8B) is shortest in the combination of F₁ and G₁ (No. 1) . On the other hand, the distance equivalent to A is longest in the combination of F₅ and G₃ (No. 15).
Further, as for the combinations of the dental drills D₁, D₂ with the spacers S₁, S₂, S₃, looking at the distance from the front end of the drill to the spacer (the distance equivalent to the distance shown by "B" in Fig. 8A) in a case of attaching one of the spacers, and looking at the distance from the front end to the protrusion portion of the drill (i.e. L₁ or L₂ shown in Fig. 4) in a case of not attaching the spacer, the distance is shortest in the combination of the first dental drill D₁ and the spacer S₃, and is L₁-3 ·p; and on the other hand, the distance is longest when the second dental drill D₂ is not attached with any of the spacers, and is L₂.

Accordingly in the present embodiment, a combination of D₁ and S₃ is set for the combination of F₁ and G₁ in which the distance from the front end of the artificial tooth root to be embedded to the upper surface of the surgical guide is shortest, as in No. 1 of Table 1. That is, the length L₁ of the first dental drill D₁ in the present embodiment shown in Fig. 4 is set to be: T+N₁+M₁+3 ·p.
On the other hand, an arrangement is made as in No. 15 of Table 1 that D₂ is not attached with a spacer, for the combination of F₅ and G₃ in which the distance from the front end of the artificial tooth root to be embedded to the upper surface of the surgical guide is longest. That is, the length L₂ of the second dental drill D₂ in the present embodiment shown in Fig. 4 is set to be: T+N₃+M₅=T+N₁+M₅+₆ ·p.
Thereby, the two dental drills D₁, D₂ and the three spacers S₁, S₂, S₃ can handle all of the 15 patterns of the combinations. Further explanations will be given below.

No. 1 is the combination of F₁ and G₁ as mentioned above, and the distance equivalent to the distance shown by A in Fig. 8B (the distance from the upper surface of the surgical guide to the front end of the artificial tooth root) is the shortest. For this, D₁ and S₃ may be combined. More specifically, the distance equivalent to the distance shown by B in Fig. 8A (the distance from the front end of the dental drill to the spacer) is L₁-3 ·p. On the other hand, the second dental drill D₂ cannot handle this case with any of the spacers.
No. 2 is a combination of F₁ and G₂, in which the distance equivalent to the distance shown by A in Fig. 8B is longer than that in the combination of F₁ and G₁ by a degree of p. With regard to this, D₁ and S₂ may be combined. More specifically, the distance equivalent to the distance shown by B in Fig. 8A is L₁-2 ·p. On the other hand, the second dental drill D₂ cannot handle this case with any of the spacers.
No. 3 is a combination of F₁ and G₃, in which the distance equivalent to the distance shown by A in Fig. 8B is longer than that in the combination of F₁ and G₁ by a degree of 2 ·p. With regard to this, D₁ and S₁ may be combined. More specifically, the distance equivalent to the distance shown by B in Fig. 8A is L₁-p. On the other hand, the second dental drill D₂ cannot handle this case with any of the spacers.

No. 4 is a combination of F₂ and G₁, in which the distance equivalent to the distance shown by A in Fig. 8B is longer than that in the combination of F₁ and G₁ by a degree of p. With regard to this, D₁ and S₂ may be combined. More specifically, the distance equivalent to the distance shown by B in Fig. 8A is L₁-2 ·p. On the other hand, the second dental drill D₂ cannot handle this case with any of the spacers.
No. 5 is a combination of F₂ and G₂, in which the distance equivalent to the distance shown by A in Fig. 8B is longer than that in the combination of F₁ and G₁ by a degree of 2 ·p. With regard to this, D₁ and S₁ may be combined. More specifically, the distance equivalent to the distance shown by B in Fig. 8A is L₁-p. On the other hand, the second dental drill D₂ cannot handle this case with any of the spacers.
No. 6 is a combination of F₂ and G₃, in which the distance equivalent to the distance shown by A in Fig. 8B is longer than that in the combination of F₁ and G₁ by a degree of 3 ·p. With regard to this, only D₁ may be used. More specifically, the distance equivalent to the distance shown by B in Fig. 8A is L₁. In this case, the protrusion portion 34b serves as a stopper. Alternatively, D₂ and S₃ may be combined. More specifically, the distance equivalent to the distance shown by B in Fig. 8A is L₂-3 ·p.

No. 7 is a combination of F₃ and G₁, in which the distance equivalent to the distance shown by A in Fig. 8B is longer than that in the combination of F₁ and G₁ by a degree of 2 ·p. With regard to this, D₁ and S₁ may be combined. More specifically, the distance equivalent to the distance shown by B in Fig. 8A is L₁-p. On the other hand, the second dental drill D₂ cannot handle this case with any of the spacers.
No. 8 is a combination of F₃ and G₂, in which the distance equivalent to the distance shown by A in Fig. 8B is longer than that in the combination of F₁ and G₁ by a degree of 3 ·p. With regard to this, only D₁ may be used. More specifically, the distance equivalent to the distance shown by B in Fig. 8A is L₁. Alternatively, D₂ and S₃ may be combined. More specifically, the distance equivalent to the distance shown by B in Fig. 8A is L₂-3 ·p.
No. 9 is a combination of F₃ and G₃, in which the distance equivalent to the distance shown by A in Fig. 8B is longer than that in the combination of F₁ and G₁ by a degree of 4 ·p. D₁ cannot handle this case with any of the spacers. On the other hand, D₂ and S₂ may be combined. More specifically, the distance equivalent to the distance shown by B in Fig. 8A is L₂-2 ·p.

No. 10 is a combination of F₄ and G₁, in which the distance equivalent to the distance shown by A in Fig. 8B is longer than that in the combination of F₁ and G₁ by a degree of 3 ·p. With regard to this, only D₁ may be used. More specifically, the distance equivalent to the distance shown by B in Fig. 8A is L₁. In this case, the protrusion portion 34b serves as a stopper. Alternatively, D₂ and S₃ may be combined. More specifically, the distance equivalent to the distance shown by B in Fig. 8A is L₂-3 ·p.
No. 11 is a combination of F₄ and G₂, in which the distance equivalent to the distance shown by A in Fig. 8B is longer than that in the combination of F₁ and G₁ by a degree of 4 ·p. D₁ cannot handle this case with any of the spacers. On the other hand, D₂ and S₂ may be combined. More specifically, the distance equivalent to the distance shown by B in Fig. 8A is L₂-2 ·p.
No. 12 is a combination of F₄ and G₃, in which the distance equivalent to the distance shown by A in Fig. 8B is longer than that in the combination of F₁ and G₁ by a degree of 5 ·p. D₁ cannot handle this case with any of the spacers. On the other hand, D₂ and S₁ may be combined. More specifically, the distance equivalent to the distance shown by B in Fig. 8A is L₂-p.

No. 13 is a combination of F₅ and G₁, in which the distance equivalent to the distance shown by A in Fig. 8B is longer than that in the combination of F₁ and G₁ by a degree of 4 ·p. D₁ cannot handle this case with any of the spacers. On the other hand, D₂ and S₂ may be combined. More specifically, the distance equivalent to the distance shown by B in Fig. 8A is L₂-2 ·p.
No. 14 is a combination of F₅ and G₂, in which the distance equivalent to the distance shown by A in Fig. 8B is longer than that in the combination of F₁ and G₁ by a degree of 5 ·p. D₁ cannot handle this case with any of the spacers. On the other hand, D₂ and S₁ may be combined. More specifically, the distance equivalent to the distance shown by B in Fig. 8A is L₂-p.
No. 15 is a combination of F₅ and G₃, in which the distance equivalent to the distance shown by A in Fig. 8B is longer than that in the combination of F₁ and G₁ by a degree of 6 ·p. D₁ cannot handle this case with any of the spacers. On the other hand, D₂ may be used without a spacer. More specifically, the distance equivalent to the distance shown by B in Fig. 8A is L₂. As described above, L₂ is set for this distance. In this case, the protrusion portion 35b serves as a stopper.

As described above, in the present embodiment, the two kinds of dental drills 34, 35, which are D₁ D₂, and the three kinds of spacers 31 to 33, which are S₁ to S₃, can be used to bore a proper hole and to regulate the depth of the hole to be bored, with respect to the combinations of the five kinds of artificial tooth roots 41 to 45, which are F₁ to F₅, with the three kinds of surgical guides 51 to 53, which are G₁ to G₃.

Table 2 contains the same items as those in Table 1 and shows a case in which the spacers included are of two kinds that are spacers S₁ and S₂.

**[Table 2]**

| No. | Artificial tooth root | Surgical guide | Distance from the upper surface of the surgical guide to the deepest part of the hole to be bored | Relation with F₁G₁ | Case of using a dental drill D 1 | | | Case of using a dental drill D 2 | | |
|---|---|---|---|---|---|---|---|---|---|---|
| | | | | | Dental drill | Spacer | Distance from the front end of the dental drill to the lower surface of the spacer | Dental drill | Spacer | Distance from the front end of the dental drill to the lower surface of the spacer |
| 1 | F₁ | G₁ | T+N ₁+M₁ | - | D₁ | S₂ | L₁-2 ·p | X | | |
| 2 | F₁ | G₂ | T+N ₂+M₁ | (F₁G₁)+p | D₁ | S₁ | L₁-p | X | | |
| 3 | F₁ | G₃ | T+N ₃+M₁ | (F₁G₁)+2 ·p | D₁ | None | L₁ | X | | |
| 4 | F₂ | G₁ | T+N ₁+M₂ | (F₁G₁)+p | D₁ | S₁ | L₁-p | X | | |
| 5 | F₂ | G₂ | T+N₂+M₂ | (F₁G₁)+2 ·p | D₁ | None | L₁ | X | | |
| 6 | F₂ | G₃ | T+N₃+M₂ | (F₁G₁)+3 ·p | X | | | X | | |
| 7 | F₃ | G₁ | T+N ₁+M₃ | (F₁G₁)+2 ·p | D₁ | None | L₁ | X | | |
| 8 | F₃ | G₂ | T+N ₂+M₃ | (F₁G₁)+3 ·p | X | | | X | | |
| 9 | F₃ | G₃ | T+N ₃+M₃ | (F₁G₁)+4 ·p | X | | | D₂ | S₂ | L₂-2 ·p |
| 10 | F₄ | G₁ | T+N ₁+M₄ | (F₁G₁)+3 ·p | X | | | X | | |
| 11 | F₄ | G₂ | T+N ₂+M₄ | (F₁G₁)+4 ·p | X | | | D₂ | S₂ | L₂-2 ·p |
| 12 | F₄ | G₃ | T+N ₃+M₄ | (F₁G₁)+5 ·p | X | | | D₂ | S₁ | L₂-p |
| 13 | F₅ | G₁ | T+N ₁+M₅ | (F₁G₁)+4 ·p | X | | | D₂ | S₂ | L₂-2 ·p |
| 14 | F₅ | G₂ | T+N ₂+M₅ | (F₁G₁)+5 ·p | X | | | D₂ | S₁ | L₂-p |
| 15 | F₅ | G₃ | T+N ₃+M₅ | (F₁G₁)+6 ·p | X | | | D₂ | None | L₂ |

As can be understood from Table 2, when the spacers included are of two kinds that are spacers S₁ and S₂, neither D₁ nor D₂ can bore a hole in the cases of Nos. 6, 8, and 10. Therefore, in the present embodiment, at least S₁, S₂. and S₃ are needed for D₁ and D₂.

Based on the above view, a system for embedding an artificial tooth root can be configured in the following way.
A group of artificial tooth roots assumed is constituted by: an artificial tooth root (e.g. F₁) which has a length of M₁ from its front end to its portion to be positioned at the face of the alveolar bone when embedded; and artificial tooth roots (e.g. F₂ to F₅) which have a length represented by Mₓ=M₁+(x-1) ·p from their front end to their portion to be positioned at the face of the alveolar bone when embedded, with an integer in a range of 2≤x≤5, thereby comprising x kinds of artificial tooth roots of M₁ to Mₓ (e.g. M₁ to M₅).

Further, a group of surgical guides assumed comprises, with an integer in a range of 1≤y≤3, y kinds of surgical guides (e.g. G₁ to G₃) of N₁ to N_{y} (e.g. N₁ to N₃) having a space of N_{y}=N₁+(y-1) ·p between the surgical guide and the face of the alveolar bone to be embedded with the artificial tooth root, in a posture of being fitted inside the oral cavity.

For these groups of artificial tooth roots and of surgical guides, a group of spacers comprises z kinds of spacers (e.g. S₁ to S₃) of Q₁ to Q_{z} provided with a spacer portion having a length of Q_{z}=z ·p in the axial direction of the tubular-shaped spacer, in a range of 1≤z≤3; the number z is defined from x and y as: z=3 in a case of x+y=8; z=2 in a case of 6≤x+y≤7; and z=1 in a case of 2≤x+y≤5.

When a distance between a face of the surgical guide which faces the alveolar bone face and its face opposite thereto is defined as T, a distance L₁ is L₁=T+N₁+M₁+z ·p from the front end to the protrusion portion of one of the two kinds of dental drills (e.g. D₁), and a distance L₂ is L₂=T+N_{y}+Mₓ from the front end to the protrusion portion of the other dental drill (e.g. D₂).

Applying the above described embodiment to this for example, since there are five kinds of artificial tooth roots assumed, x is 5 (x=5) ; and since there are three kinds of surgical guides assumed, y is 3 (y=3). Accordingly, x+y=8 can be derived, and at least three spacers are needed, matching Table 1. At this time, the L₁ of the dental drill D₁ may be L₁=T+N₁+M₁+3 ·p; and the L₂ of the dental drill D₂ may be L₂=T+N₃+M₅.

In addition, a case of configuring a system for embedding an artificial tooth root with three kinds of artificial tooth roots assumed and two kinds of surgical guides assumed will be considered as another example. At this time, since x is 3 (x=3) and y is 2 (y=2), x+y is 5 (x+y=5); accordingly, S₁ is the only spacer needed. In this case, the L₁ of the dental drill D₁ may be L₁=T+N₁+M₁+p; and the L₂ of the dental drill D₂ may be L₂=T+N₂+M₃.

With a system for embedding an artificial tooth root such as this, it is possible to bore holes of various depths into the alveolar bone more stably in accordance with the combinations of the kinds of artificial tooth roots assumed and the kinds of the surgical guides assumed, without including more dental drills and spacers than needed.

Further, in providing a system for embedding an artificial tooth root, it is preferable to provide a table (such as Table 1) that shows not only the dental drills and the spacers but also how these can be combined so as to accord with the combinations of the artificial tooth root and the surgical guide.

In the above described example, there has been introduced a system for embedding an artificial tooth root including all of the artificial tooth roots, surgical guides, and spacers which can be obtained based on that concept. However, as long as the system is configured based on the above concept, it may exclude some of them. According to this, the combination which is included in the system for embedding an artificial tooth root based on the above concept but is rarely employed in an actual situation may be excluded, thereby enabling a system for embedding an artificial tooth root to be a more compact one.

Further, with the above concept taken into account, the actual circumstances in which a system for embedding an artificial tooth root will be applied may also be considered. That is, taking it into account that a dental drill will be inserted into the oral cavity of a human to practice procedures, a system for embedding an artificial tooth root may be configured such that the upper limit of the size of the L₂ of the longer dental drill is 30 mm, having L₂-L₁=2 ·p from a viewpoint that the kinds of artificial tooth root differing in length are limited to some extent. At this time, a system for embedding an artificial tooth root such as above may be constituted in a range that meets these conditions.

### Description of the Reference Numerals

- 30: system for embedding artificial tooth root
- 31: first spacer
- 32: second spacer
- 33: third spacer
- 34: first dental drill
- 34a: drill main body
- 34b: protrusion portion
- 35: second dental drill
- 35a: drill main body
- 35b: protrusion portion
- 40: group of artificial tooth roots
- 41-45: artificial tooth root
- 50: group of surgical guides
- 51-53: surgical guide

## Claims

1. A set of dental components for embedding an artificial tooth root, comprising two kinds of dental drills to bore a hole into an alveolar bone, and a group of spacers comprising one to three kinds of spacers to be attached to said dental drills, for combinations of an artificial tooth root and a surgical guide, said artificial tooth root being selected from a group of artificial tooth roots formed of two to five kinds of artificial tooth roots, and said surgical guide being selected from a group of surgical guides formed of one to three kinds of surgical guides,
wherein, with an integer in a range of 2≤x≤5, said group of artificial tooth roots assumed is constituted by an artificial tooth root which has a length of M₁ from its front end to its portion to be positioned at the face of the alveolar bone when embedded, and an artificial tooth root which has a length represented by Mₓ=M₁+ (x-1) ·p from its front end to its portion to be positioned at the face of the alveolar bone when embedded, thereby comprising x kinds of artificial tooth roots of M₁ to Mₓ;
with an integer in a range of 1≤y≤3, said group of surgical guides assumed comprises y kinds of surgical guides of N₁ to N_{y} having a space of N_{y}=N₁+(y-1) ·p from the surgical guide to the face of the alveolar bone to be embedded with said artificial tooth root, in a posture of being fitted in an oral cavity;
said two kinds of dental drills each comprises a stick-shaped drill main body provided with a drill blade, and a protrusion portion arranged in a manner protruding from one part of the side face of said drill main body;
said spacer is in a tubular shape, allowing said drill main body to penetrate inside the tubular shape and not allowing the portion of the drill provided with said protrusion portion to penetrate thereinside;
with an integer in a range of 1≤z≤3, a group of said spacers comprises z kinds of spacers of Q₁ to Q_{z} provided with a spacer portion having a length of Q_{z}=z ·p in an axial direction of the tubular-shaped spacer; the number z is defined from said x and said y as:
z=3 in a case of x+y=8;
z=2 in a case of 6≤x+y≤7; and
z=1 in a case of 2≤x+y≤5; and
said two kinds of dental drills having lengths differing from each other, and when a distance between a face of said surgical guide which faces said alveolar bone face and its face opposite thereto is defined as T, a distance L₁ is L₁=T+N₁+M₁+z ·p from the front end to said protrusion portion of one of said two kinds of dental drills, and a distance L₂ is L₂=T+N_{y}+Mₓ from the front end to said protrusion portion of the other dental drill.

2. The set of dental components for embedding an artificial tooth root according to claim 1, wherein said L₂ is 30 mm or less; and
a relation between said L₂ and said L₁ is configured in a range satisfying L₂-L₁=2 ·p.
